# EUROPEAN PATENT SPECIFICATION

(11) **EP 2 865 421 B1**
(45) Date of publication and mention of the grant of the patent: **30.05.2018**
(21) Application number: 13190536.6
(22) Date of filing: 28.10.2013
(51) Int. Cl.: A61P 17/00, A61P 27/02, A61P 11/02, A61K 31/35, A61K 31/351, A61K 31/4453, A61K 36/76

(54) **Bitter taste receptor agonists for topical use**
Bitterstoffe für die topische Verabreichung
Composés amers pour l'administration topique

(43) Date of publication of application: 29.04.2015
(73) Proprietor: Albert-Ludwigs-Universität Freiburg, 79085 Freiburg (DE)
(72) Inventor: Schempp, Christoph, 79102 Freiburg (DE); Woelfle, Ute, 79108 Freiburg (DE)
(74) Representative: Kalhammer, Georg

(56) References cited:
- EP-A1- 2 735 302
- WO-A1-2013/112865
- WO-A1-2013/158661
- WO-A2-2012/021291
- JP-A- 2010 229 124
- ROBERT J. LEE ET AL: "T2R38 taste receptor polymorphisms underlie susceptibility to upper respiratory infection", JOURNAL OF CLINICAL INVESTIGATION, vol. 122, no. 11, 8 October 2012 (2012-10-08), pages 4145-4159, XP055092197, ISSN: 0021-9738, DOI: 10.1172/JCI64240
- DATABASE WPI Week 201049, Derwent Publications Ltd., London, GB; AN 2009-S44830 & KR 2009 0 126 881 A (AMOREPACIFIC CORP) 09 December 2009
- DATABASE WPI Week 200454, Derwent Publications Ltd., London, GB; AN 2004-557401 & JP 2004 210656 A (DAICHO KIKAKU YG) 29 July 2004

## Description

### FIELD OF THE INVENTION

The present invention relates to a bitter taste receptor agonist and its pharmaceutical application.

### BACKGROUND OF THE INVENTION

Sweet and bitter taste receptors are G-protein-coupled receptors (GPCRs) that are found in insects (drosophila) as well as in vertebrates from fish to man [1]. The GPCR T1R3 contains an evolutionary conserved gene sequence and is associated with sweet and umami (glutamate) taste sensation [2] whereas the T2R gene family is very divers [3] [4] [5] and responsible for bitter tasting. The general hypothesis is that the flavor of bitter prevents us from swallowing potential toxic substances, while sweet and umami taste encourage us to eat food containing carbohydrates and amino acids [6]. Toxic bitter compounds have very different molecular shapes and sizes, so that it is not surprising that the receptors that recognize them are diverse. Although bitter taste is seen as a code for danger many bitter compounds also provide important nutrients [7]. The signaling cascade of bitter as well as sweet taste receptors lead via the G-protein α-gustducin to the activation of phospholipase C-β2 (PLCβ2) and the formation of inositoltriphosphate (IP₃) as well as diacylglycerol (DAG). These second messengers lead to the release of internal calcium and subsequent activation of transient receptor potential cation channel 5 (TRPM5). Activation of taste receptor cells by a stimulus is then transported via afferent neurons and processed in taste centers in the brain [8]. Among these proteins that are involved in taste transduction (sweet, bitter, umami) α-gustducin has been first directly linked to taste transduction in KO mouse models [8] and is still widely used as a marker for chemosensory cells throughout the body [6], although α-gustducin KO mice still have a slight bitter/sweet taste sensation.

The bitter taste receptor T2R38 is particularly well studied and largely responsible for human polymorphism in tasting phenylthiocarbamide (PTC). Many previous studies have demonstrated that for people with the T2R38 taster form concentrations of PTC are intensely bitter that are neutral for those with the non-taster form [9] [10] . The difference between the taster T2R38 protein and non-taster form lies in the amino acid residues at position 49, 262 and 296. The functional allele of the receptor contains a prolin, alanine and valine (PAV), while the nonfunctional allele contains an alanine, valine and isoleucine (AVI) at these positions respectively [11]. Based on bioinformatics and modeling, it has been suggested that the valine at the third position in the polymorphism is involved in the interhelical hydrogen bonding that stabilizes the receptor structure and allows remarkable receptor activation only in tasters [12]. Balancing selection maintains probably both taster and non-taster T2R38 alleles in human populations.

Furthermore it was shown that bitter taste receptors are expressed in cell types outside the gustatory epithelium of the tongue and oropharynx. They influence glucose homeostasis in the human gastrointestinal tract [14] and in bronchial smooth muscle cells where they stimulate the relaxation of airways for improved breathing [15],[16]. Most experiments in this field were performed in mice or rats. However, a comparison between human and rodent T2R genes indicates that rodents possess over 40% more bitter taste receptors (25 T2Rs versus 36 T2Rs) and probably a wider spectrum of bitter sense detection (Chandrashekar et al. 2000) compared to humans. Heterologous expression assays demonstrate that human T2R16 binds the bitter glucopyranoside salicin, whereas mice don't taste salicin. Furthermore T2Rs can be detected in cultured human airway cells, but not in the lower airway of mice. This discrepancy could represent species difference or a difference of the *in vivo* (mouse) and *in vitro* (human) situation [6]. Therefore it is important to study the expression of human T2Rs in extra oral tissues in order to elucidate their normal physiological roles outside the gustatory system. In situ tissue stainings are required to determine specific T2R expressing cell types in addition to PCR analyses to determine the protein distribution in the natural cellular environment.

In the present invention the expression of bitter taste receptors in various human tissues was investigated by staining human tissue microarrays containing 45 tissues from healthy human donors. Two conserved bitter taste receptors (T2Rs) were selected as targets for this analysis, T2R1 and T2R38 [17]. Several tissues outside the gustatory system showed a strong bitter taste receptor expression without simultaneous sweet taste receptor expression.

Available treatment options for skin disorders or pathological conditions affecting the sinonasal mucosa show either only a weak effect or possess several side effects. Glucocorticoids for example can lead to atrophy or bleeding of the skin and mucosa. Common side effects of xylometazoline or adrenalin-derivatives are rhinitis medicamentosa, atrophy of mucosa or loss of effect.

Therefore there is a high need for new treatment options, like topical administration of bitter taste receptor ligands. Bitter taste receptors are inter alia expressed in keratinocytes (skin cells) and immune cells (mast cells). Ligands to these receptors induce a significant calcium influx which leads to reduced release of histamine, proteases and proinflammatory cytokines in mast cells. In skin cells the expression of differentiation markers is stimulated resulting in a stronger skin- and mucosa barrier.

The inventors of this application surprisingly found, that the topical application of a bitter taste ligand containing cream relieved itchiness and redness in a case of atopic eczema immediately after the first application.

WO 2013/112865 A1 discloses a method for treating a respiratory infection in a subject, the method comprising administering a composition to the respiratory tract of the subject, wherein the composition comprises an agent capable of activating a bitter taste signal pathway or inhibiting a sweet taste signal pathway in the subject, thereby treating the respiratory infection in the subject.

JP2010/229124 A provides a solution which contains diphenhydramine or a salt thereof, an organic acid and a plasticizer and preferably furthermore caramel, is good in compliance on administration, hardly causes the softening and cracking of capsule skin membranes, and is filled into capsules, and to provide a capsule filled with the solution. The solution is disclosed as useful for the treatment of i.a. allergic or acute rhinitis, pruritus, urticaria and dermatological disorders. KR 2009 0126881 A discloses a slimming patch containing caffeine to effectively remove cellulite and help in making slim body line that contains 0.01-4 weight% of caffeine as an active ingredient, 0.1-20 weight% of glyceryl monolaurate as a skin permeation enhancer, theanine, genistein, L-carnitine, coffee extract (Coffea Arabica (Coffee Seed) Bean Extract), Ilex Paraguariensis (Leaf) extract, and Citrus Aurantium Amara (Bitter Orange) Flower Extract, has thickness of 10-200 um and is used in the form of plaster formulation on site having subcutaneous fat such as buttock, rear side of thigh.

JP2004/210656 A discloses a preparation for skin, characterized by comprising isoflavone and/or an acylisoflavone and/or an isoflavone glycoside and a pungent taste substance, bitter taste substance or sour taste substance and not containing cholic acid, scymnol and a scymnol ester.

EP2735302 A1 discloses a topical composition comprising a hop extract, and at least one member, selected from the group consisting of dermatologically and cosmetically acceptable carriers, excipients, and diluents; wherein the 8-prenyl naringenin (8-PN) content in the composition is between 0.1 and 1.0 % by weight, and uses of said composition.

### SUMMARY OF THE INVENTION

The present invention relates to the following embodiments (1) to (4).
(1) A ligand to a bitter taste receptor (T2R) for use in the treatment of a skin disorder, wherein the ligand is administered topically to the skin, and wherein the ligand is amarogentin.
(2) The ligand for use according to item (1), wherein the bitter taste receptor is taste receptor 2 member 1 (T2R1).
(3) The ligand for use according to item (1), wherein said skin disorder is selected from the group consisting of pruritus, urticaria, dermatitis, eczema and combinations thereof.
(4) A pharmaceutical composition for topical use in the treatment of a skin disorder, comprising a ligand to a bitter taste receptor as defined in item (1), wherein said composition is a nasal spray, an ophthalmic composition, an ointment, a lotion, a cream or a gel.

### BRIEF DESCRIPTION OF THE FIGURES

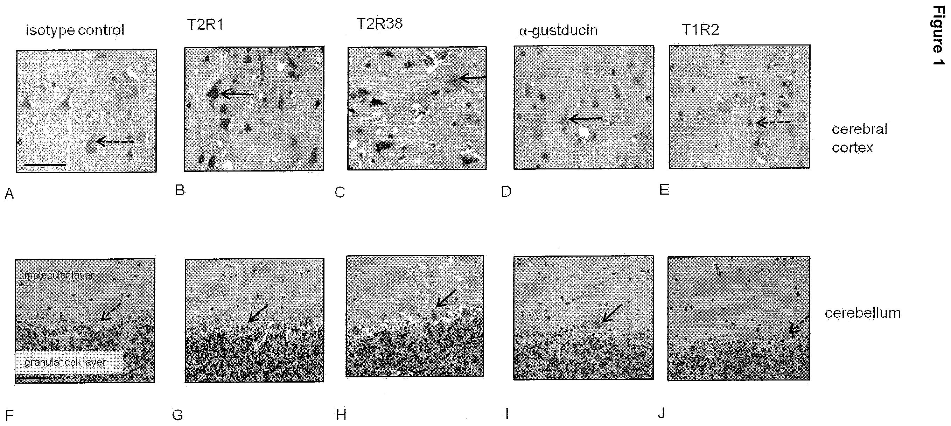

Figure 1: Staining of human brain tissue.
   Cross sections of the human tissue microarray with tissue of the cerebral cortex (A-E) and cerebellum (F-J) were stained with an isotype control (A, F) or antibodies against T2R1 (B, G), T2R38 (C, H), α-gustducin (D, I) and T1R2 (E, J). The arrow indicates pyramidal neurons (A-E) or Purkinje neurons (F-J). Arrows in red point to positively and arrows in black to negatively stained cells. The indicated bars correspond to 50 µm (A-E) and 100 µm (F-J).
Figure 2: Staining of epidermal tissue.
   Cross sections of the human tissue microarray with human skin was stained with were stained with an isotype control (A) or antibodies against T2R1 (B), T2R38 (C), α-gustducin (D) and T1R2 (E). The indicated bar corresponds to 100 µm.
Figure 3: Staining of placental tissue.
   Cross sections of the human tissue microarray with human placenta (chorionic plate) (A-E) were stained with an isotype control (A) or antibodies against T2R1 (B), T2R38 (C), α-gustducin (D) and T1R2 (E). Arrows in red point to positively and arrows in black to negatively stained cells. The indicated bar corresponds to 100 µm.

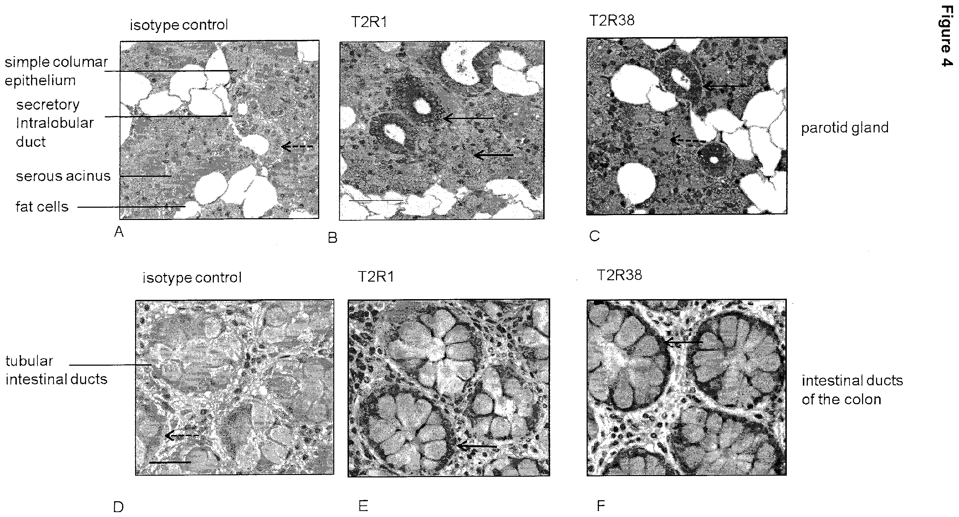

Figure 4: Staining of the parotid glands and intestinal ducts of the colon.
   Cross sections of the human tissue microarray with human parotid gland (A-C) and the ducts of the colon (D-F) were stained with an isotype control (A ,D) or with antibodies against T2R1 (B, E) and T2R38 (C, F). Arrows in red point to positively and arrows in black to negatively stained cells. The indicated bar corresponds to 50 µm.

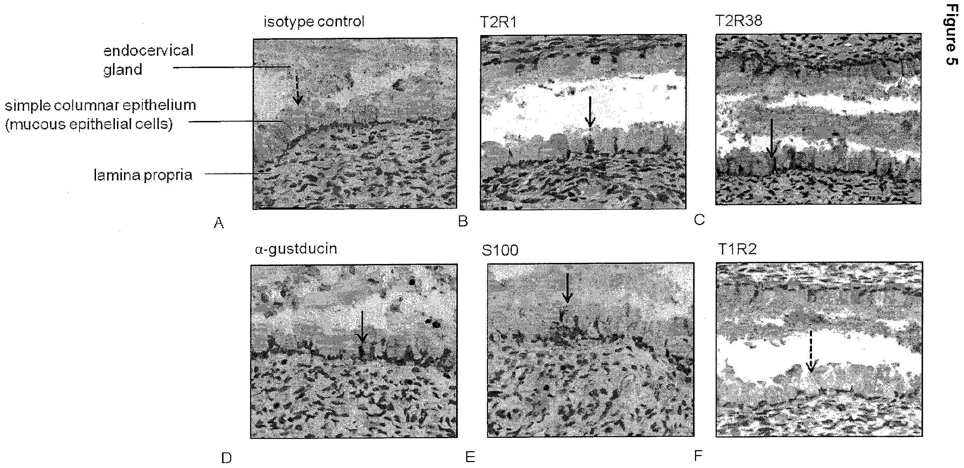

Figure 5: Staining of the endocervix.
   Cross sections of the human tissue of endocervix (A-F) was stained with an isotype control (A) or antibodies against T2R1 (B), T2R38 (C), α-gustducin (D), S100 (E) and T1R2 (F). Arrows indicate nuclei of possible dentritic cells in the ducts. Arrows in red point to positively and arrows in black to negatively stained cells. The indicated bar corresponds to 50 µm.

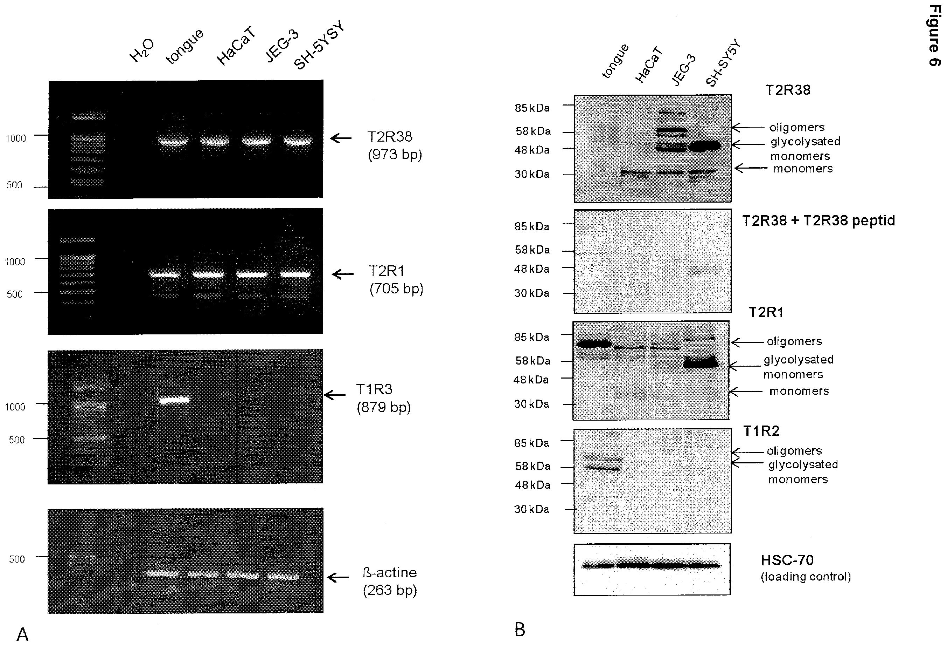

Figure 6: Gene and protein expression of T2R1, T2R38 and T1R3 in tongue tissue and human cell lines.
   RT-PCR with RNA from the selected cell lines (the human neuroblastoma cell line SH-SY5Y, the human placental cell line JEG-3 and the human keratinocyte cell line HaCaT) and commercially available tongue RNA was performed for T2R1, T2R38, T1R3 and the house keeping gene ß-actine. The H₂O control contains H₂O instead of the template. The arrows point to the PCR product (A). Western blot was performed with protein lysates of HaCaT, JEG-3 and SH-SY5Y cells and of tongue tissue visualized by staining with anti-T2R1, anti-T2R38, anti- T1R2 and the anti-HSC-70 (housekeeping gene) antibody. The bands correspond to the predicted molecular weight of receptor monomers, glycosylated monomers and oligomers. Pre-absorption of the T2R38 antibody with the corresponding antigenic peptides (T2R38 peptide) for 1h at 37°C nearly totally abolished the signal with the anti-T2R38 antibodies. The size of the marker proteins in kDa are indicated (B).

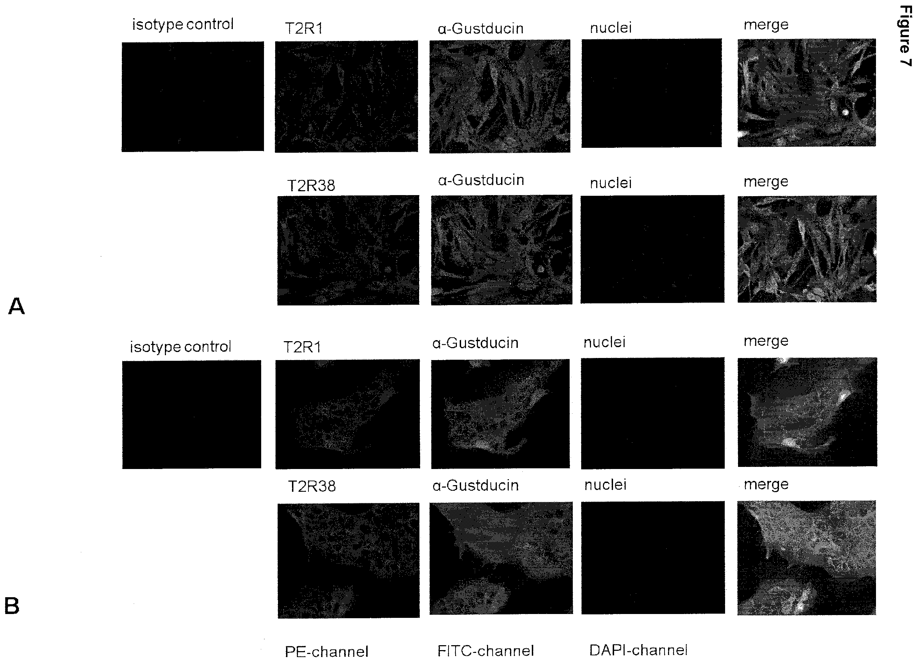

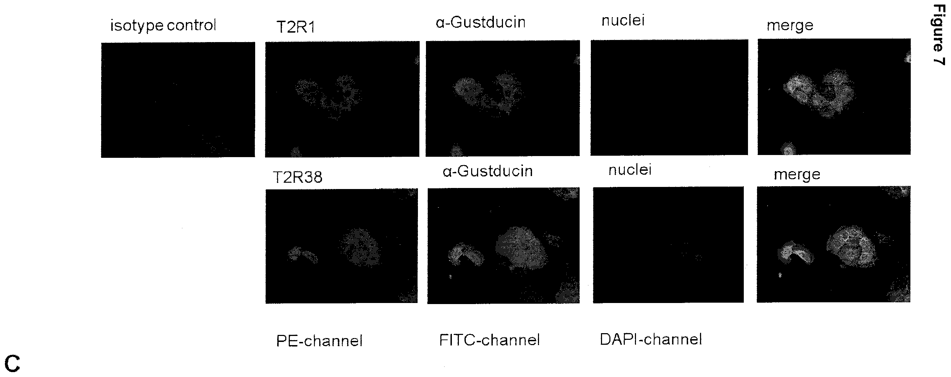

Figure7: Double staining of human cell lines with T2R1 or T2R38 and α-gustducin.
   The selected cell lines were the human neuroblastoma cell line SH-SY5Y (A), the human placental cell line JEG-3 (B) and the human keratinocyte cell line HaCaT (C). The rabbit human anti-T2R1 and rabbit human anti-T2R38 antibody was visualized by a secondary-Alexa 555- coupled antibody and the goat human anti- α-gustducin antibody by a secondary Alexa 488- coupled antibody. Overlay pictures of the merged FITC and PE channels are shown (merge). The merged images of α-gustducin and T2R1 or T2R38 expression indicated double labeling of all cells in the three tested cell lines. The isotype control showed in all cell lines no fluorescence signal. The pictures were photographed at magnification of 400x.

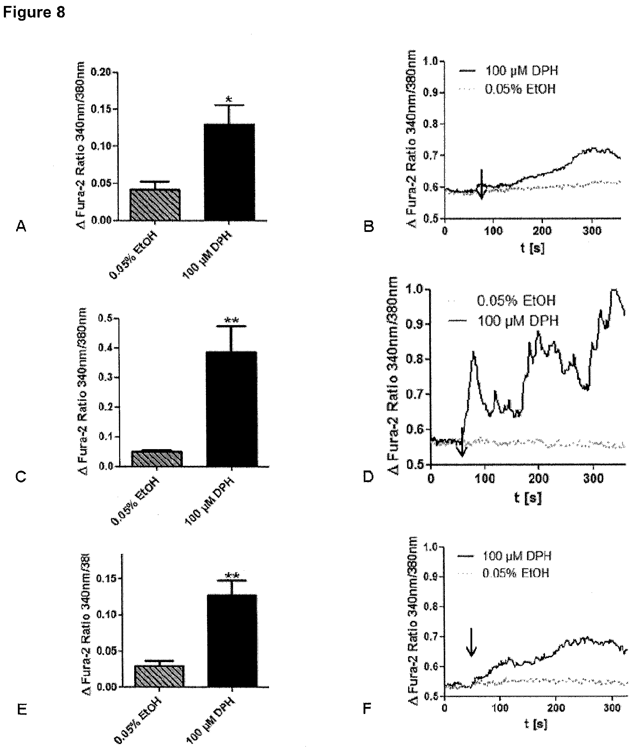

Figure 8: Diphenidol (DPH) evoked a calcium influx in SH- SY5Y neuroblastoma cells (A, B), JEG-3 placental cells (B, D) and HaCaT keratinocytes (E, F). The cells were stimulated with DPH (100 µM) or ethanol (EtOH, 0.05%) at time point 60 s as indicated with the arrow and calcium currents were analyzed by calcium imaging. Calcium influx is expressed as the difference of the fura-2 ratio before and after stimulation and is represented in bars (A, C, E) or as function of the time (B, D, F). Representative traces including 9-20 DPH-treated and 12-14 EtOH-treated cells. The data are shown with standard deviation (SEM) and were analyzed with the unpaired, two-tailed t-test (* p < 0.05; ** p < 0.01, n=5).

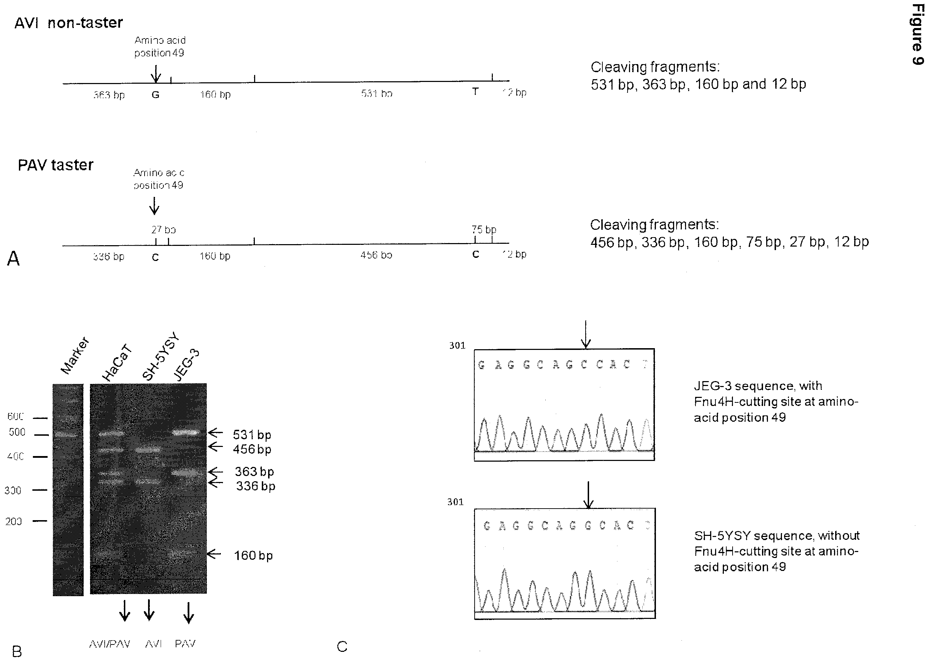

Figure 9: Determination of the T2R38 gene allele in the cell lines HaCaT, JEG-3 and SH-SY5Y to distinguish between PTC taster and PTC non-taster.
   (A) Scheme to demonstrate the cleaving products by an restriction enzyme-based analysis of PTC taster and non-taster. PTC tasters have the haplotype PAV and non-tasters have the haplotype AVI (A=Ala, V=Val, I=Ile) at the protein positions 49, 262 and 296 (B) Cleaving pattern of the cell lines HaCaT, JEG-3 and SH-SY5Y to determine the taster status. First a 1067 bp fragment of the N-terminal portion of T2R38 is amplified by PCR at an annealing temperature of 58°C. Then the PCR product was digested with Fnu4H, that cuts DNA at the sequence GCNGC (N is A, G, C or T). This sequence overlaps both sites of polymorphisms. At the amino acid position 262 and 49 PAV tasters contain a cutting site while AVI non-tasters do not. The restriction pattern of the 1 Kb TAS2R38 PCR product indicates the sequence at both polymorphic sites and was determined in a 2% agarose gel. A 557 Fnu4H fragment in non-tasters is cleaved to 456 bp and 75 bp fragments in PAV tasters. A 363 bp Fnu4H fragment in non-tasters is cleaved to 336 bp and 27 bp fragments in PAV tasters. Heterozygosity displays both the cleaved and uncleaved products. HaCaT appears to be AVI/PAV heterocygous, JEG PAV taster and SH-SY5Y non-taster. (C) Sequence of T2R38 DNA at amino acid position 49 clearly indicated JEG as PAV taster with a Fnu4H cleaving site and SH-SY5Y as PTC non-taster without cleaving site.

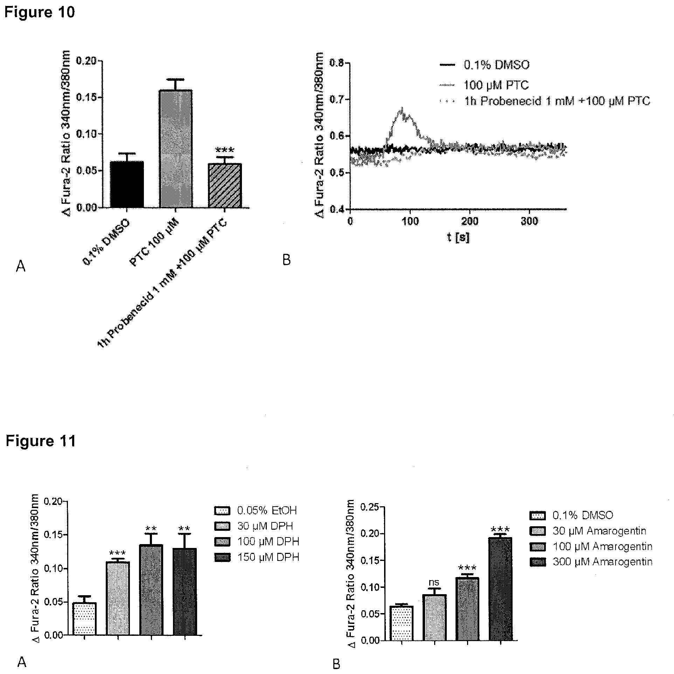

Figure 10: PTC induces calcium influx in JEG-3 cells. (A) JEG-3 cells are stimulated after 60s with PTC (100 µM) or DMSO (0.05%) as indicated with the arrow and calcium currents are analyzed by calcium imaging. Calcium influx is expressed as the difference of the fura-2 ratio before and after stimulation. 1h pre-incubation with probenecid (1 mM) reduces PTC-induced calcium currents to solvent level. Data + SEM. n = 5. t-test, unpaired, two-tailed. *** p < 0.001. (B) Representative traces including 28 (0.05% DMSO), 24 (PTC 100 µM) and 24 (1h pre-incubation with probenecid (1 mM), stimulation with PTC (100 µM)) JEG-3 cells are shown.
Figure 11: Diphenidol (DPH) and amarogentin induces calcium influx in HaCaT keratinocytes. Keratinocytes were stimulated after 60s with various concentrations of DPH (A) or amarogentin (B) compared to 0.1% EtOH. Calcium currents are analyzed by calcium imaging using the calcium-sensitive probe fura-2-am. Calcium influx is expressed as the difference of the fura-2 ratio before and after stimulation. Data + SEM. n = 6. t-test, unpaired, two-tailed. ** p < 0.01, *** p < 0.001.

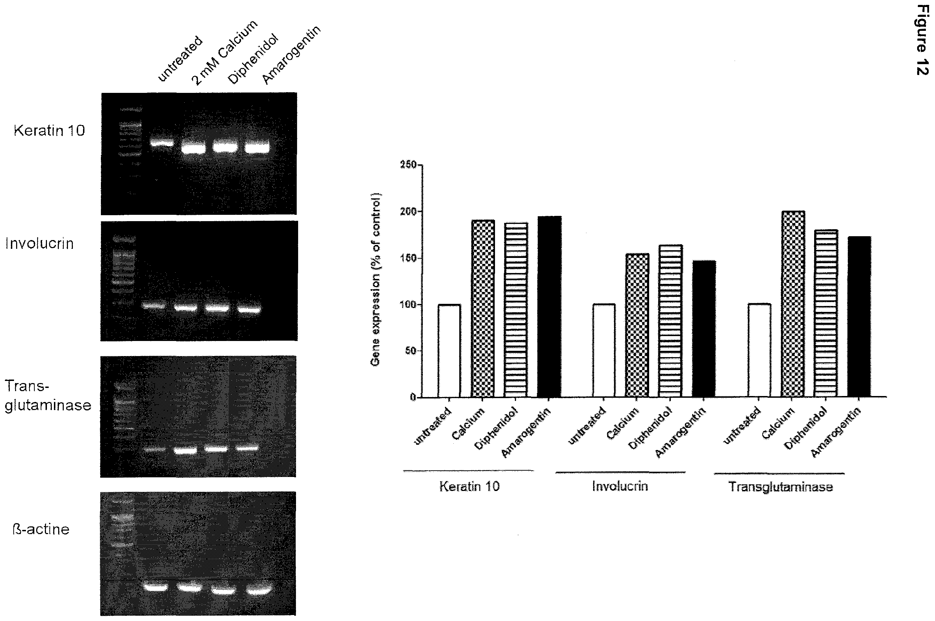

Figure 12: Effect of bitter compounds on differentiation of primary keratinocytes.
   Amarogentin and Diphendidol induced differentiation in human primary keratinocytes (hPKs). hPKs were treated with calcium (2mM) and amarogentin or diphenidol for 3 days. Total mRNA of treated hPKs was isolated, reverse transcribed, and subjected to PCR. The expression of differentiation markers in untreated and amarogentin, diphenidol or calcium (2mM) treated samples was analyzed. The histograms show relative expressing levels of differentiation markers in hPKs compared with their normalized expression levels in the untreated control cells.

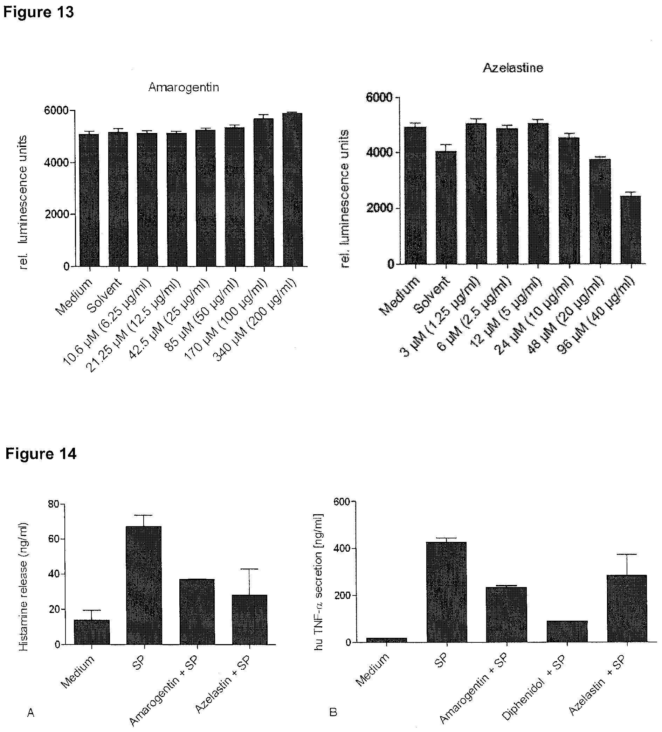

Figure 13: Amarogentin shows no cytotoxicity in HaCaT cells in the concentration range of the experiments. Cell viability was measured 24 h after amarogentin treatment.
Figure 14: Amarogentin inhibits Substance P (SP)-triggered mediator release from the human mast cell line LAD-2. LAD-2 cells were stimulated with SP (2 µM). In some experiments cells were pre-incubated with the bitter taste receptor ligand amarogentin (136 µM) or diphenidol (136 µM) or the positive control azelastine (24 µM). (A) Histamine release was measured 20 min after SP stimulation (B) TNF-α release was measured 24 h after SP stimulation.

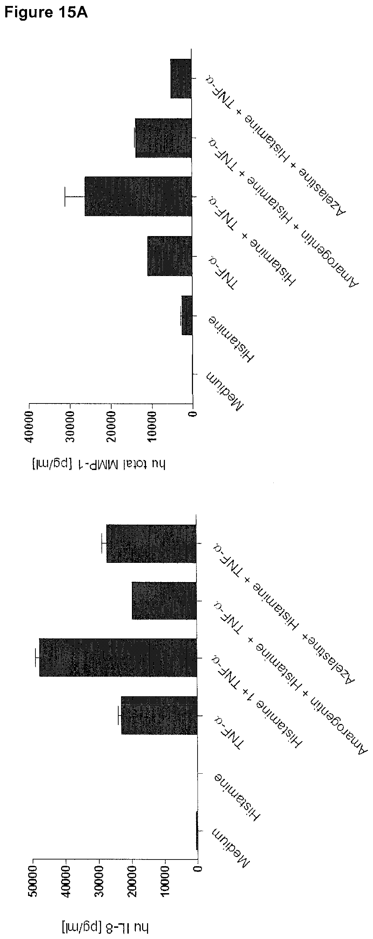

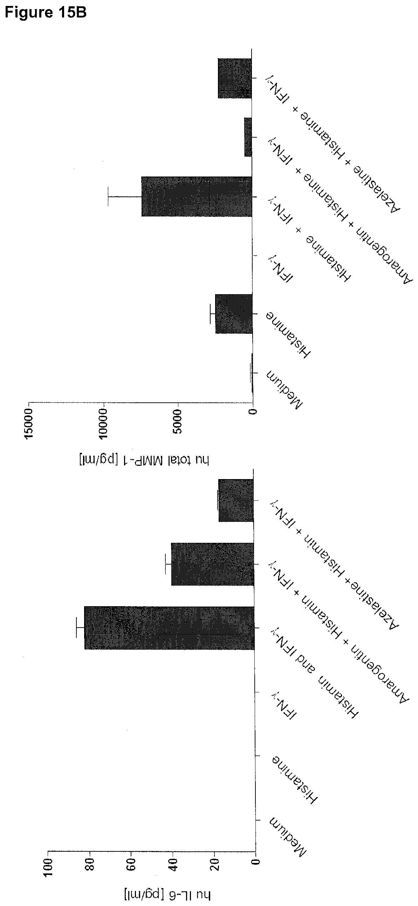

Figure 15: Amarogentin inhibits IL-6 or IL-8 and MMP1-secretion in the keratinocyte cell line HaCaT after stimulation with histamine and TNF-α or IFN-γ. (A) HaCaT cells were stimulated with histamine and TNF-α as indicated. In some experiments cells were pre-incubated with amarogentin (136 µM) or the control azelastine (24 µM) for 30 minutes. (B) HaCaT cells were stimulated with histamine and IFN-γ as indicated. In some experiments cells were pre-incubated with amarogentin (136 µM) or azelastine (24 µM).

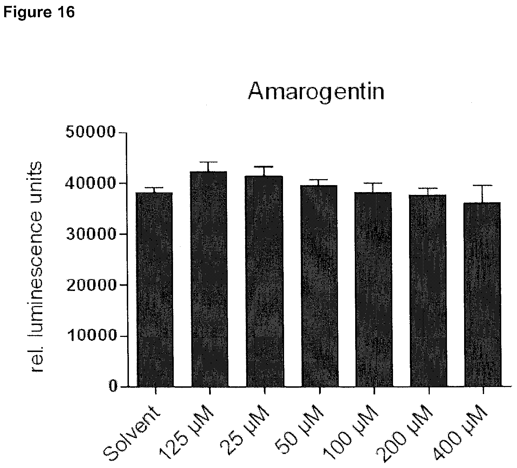

Figure 16: Amarogentin and azelastine show no cytotoxicity in LAD-2 cells in the concentration range used in the experiments. Cell viability was measured 24 h after amarogentin and azelastine treatment.

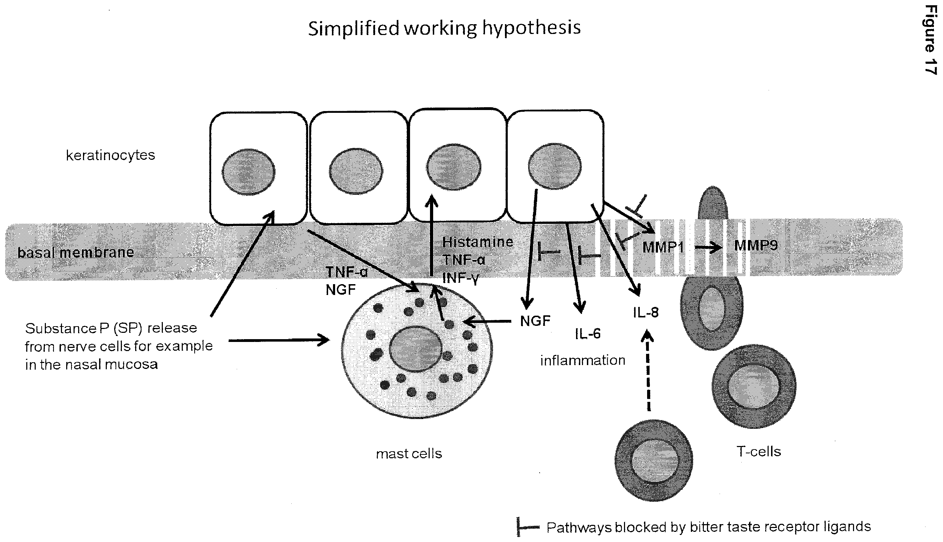

Figure 17: Simplified working hypothesis for the reaction mechanism of bitter taste receptor ligands on cellular interactions among mast cells, T cells and keratinocytes in the epidermis and uppermost dermis of inflamed skin. The reaction might be triggered by substance P (SP) release from nerve cells. In these mechanisms bitter taste receptor ligands inhibit the secretion of histamine, TNF-α and INF-γ from mast cells, so that the section of MMP-1, IL-6 and IL-8 and possibly also NGF from human keratinocytes is inhibited. A reduced MMP-1 expression results in reduced degradation of the basement membrane and a reduced T cell transmigration to the epidermis. In this way the secretion of inflammatory cytokines by T cells in the epidermis can be reduced or avoided.

### DETAILED DESCRIPTION OF THE INVENTION

### Definitions

Unless otherwise stated, the following terms used in the specification and claims have the meaning given below.

A "ligand" as used herein is intended to mean a substance that either inhibits or stimulates the activity of a receptor and/or that competes for the acceptor in a binding assay.

An "agonist" is defined as a ligand increasing the activity of a receptor. An agonist of the bitter taste receptor is a ligand increasing the activity of the bitter taste receptor.

An "antagonist" is defined as a ligand decreasing the activity of a receptor, here the bitter taste receptor, when inhibiting the action of an agonist. Antagonism usually is a property of the ligand measured in the presence of an agonist.

"Epithelial tissues" or "barriers" are used in multicellular organisms to protect areas of their bodies that come into contact with the external environment. In the intestine the epithelial barrier supports nutrient and water transport by preventing microbial contamination of the intestinal tissues. Along with plasma membranes the intercellular tight junction is the primary cellular determinant of epithelial barrier function. Disruption of tight junction structure as a result of specific protein mutations or aberrant regulatory signals can be both a cause and an effect of diseases.

A "pathological condition" is a disease or a condition involving or caused by a disease.

A "topical medication" is a medication that is applied to body surface such as the skin or mucus membranes to treat ailments via a large range of classes including but not limited to creams, foams, gels, lotions and ointments. Many topical medications are epicutaneous, meaning that they are applied directly to the skin. Topical medications may also be inhalational or applied to the surface of tissues other than the skin such as eye drops applied to the conjunctiva, or ear drops placed in the ear, or medications applied to the surface of a tooth. A topical effect in the pharmacodynamic sense may refer to a local rather than systemic target for medication. However, many topically administered drugs have systemic effects.

All concentrations indicated herein are to understood as concentration (w/w). That is, a concentration in % means %(w/w), unless indicated otherwise.

"Taste receptors" are responsible in identifying food components found in the diet of humans and animals. Basic taste qualities are sour, salty, sweet, bitter and umami. Sweet and bitter taste receptors are G protein-coupled receptors that are found in insects as well as in vertebrates. The G protein-coupled receptor T1R3 is associated with sweet and umami taste sensation. The gene family T2R is very diverse and is responsible for bitter tasting.

Examples of bitter taste receptors can be found at BitterDB (Ayana Wiener; Marina Shudler; Anat Levit; Masha Y. Niv. BitterDB: a database of bitter compounds. Nucleic Acids Res 2012, 40:D413-419.). According to this invention, the ligand can be a ligand to taste receptor 2 member 1 (T2R1).

NCBI Accession Numbers of T2R1 mRNA and protein are NM_019599.2 and NP_062545.1, respectively. NCBI Accession Numbers of T2R38 mRNA and protein are NM_176817.4 and NP_789787.4, respectively. The T2R1 receptor may comprise or consist of an amino acid sequence as shown in NCBI Accession Number NP_062545.1 (UniProt Accession Number Q9NYW7). It is preferred that the ligand is capable of activating at least one bitter taste receptor, e.g. one or more of the bitter taste receptors recited hereinabove.

Examples of bitter taste receptor ligands (bitter compounds) can be found in the database "BitterDB" (Ayana Wiener; Marina Shudler; Anat Levit; Masha Y. Niv. BitterDB: a database of bitter compounds. Nucleic Acids Res 2012, 40:D413-419.)

Methods for determining whether a given compound is a ligand to a bitter taste receptor are known to one of ordinary skill, see e.g. Jacob P. Ley, Chem. Percept. (2008) 1: 58-77. According to the present invention, the method described in Meyerhof, Chem. Senses (2010) 35: 157-170, can be used. According to this invention, the ligand to a bitter taste receptor is a bitter taste receptor agonist.

According to the present invention the ligand to a bitter taste receptor is amarogentin.

In one embodiment, the ligand is the ligand to T2R1 and is amarogentin.

### Pharmaceutical compositions

The ligand of the present invention or pharmacologically acceptable salts thereof according to the invention may be formulated into pharmaceutical compositions or various dosage forms. To prepare the pharmaceutical composition of the invention, one or more compounds including optical isomers, enantiomers, diastereomers, racemates or stereochemical mixtures thereof, or pharmaceutically acceptable salts thereof as their active ingredient is mixed with appropriate carriers and additives according to techniques known to those skilled in the art.

A pharmaceutically acceptable salt refers to a salt form of the compounds of the present invention in order to permit their use or formulation as pharmaceuticals and which retains the biological effectiveness of the free acids and bases of the specified compound that is not biologically or otherwise undesirable. Examples of such salts are described in the "Handbook of Pharmaceutical Salts: Properties, Selection and Use", Wermuth, C.G. and Stahl, P.H. (eds.), Wiley Verlag, Helvetica Acta, Zurich, 2002, [ISBN 3906390-26-8].

The concentration of the ligand in the pharmaceutical composition of the present invention may range from 0.001 to 10 %, preferably from 0.01 to 5%, more preferably from 0.1% to 1% (w/w).
If the pharmaceutical composition is a cream or ointment, the concentration may range from 0.001 to 10 %, preferably from 0.01 to 5%, more preferably from 0.1% to 2% (w/w).

For amarogentin the concentration in the pharmaceutical composition may range from 0.001 to 3%, preferably from 0.01 to 2% more preferably from 0.1 to 1% (w/w).
If the pharmaceutical composition is a cream, the concentration of amarogentin may range from 0.001 to 3%, preferably from 0.01 to 2% more preferably from 0.1 to 1% (w/w).

The carriers and additives used for such pharmaceutical compositions can take a variety of forms depending on their anticipated mode of administration. Compositions for liquid preparation include solutions, emulsions, dispersions, suspensions, syrups, elixirs and the like with suitable carriers and additives being water, alcohols, oils, glycols, preservatives, flavoring agents, coloring agents, suspending agents and the like. The pharmaceutical compositions according to the embodiments of the present invention include those suitable for topical (i.e. skin) and transdermal administration. In a preferred embodiment of the present invention the pharmaceutical composition is an ointment, a lotion, a cream or a gel.

Antimicrobial agents and/or preservatives may be also present in this composition.

Four formulations are described in the following.

### 1% salicin cream

1.0 g salicin
6.0 g cetyl alkohol
4.0 g glycerol mono stearate 60
7.5 g medium-chain triglycerides
25.5 g white vaseline
7.0 g macrogol-1000- glycerol mono stearate
10.0 g propylene glycole
39.0 g purified water

### 5% willow bark extract cream

5.0 g willow bark extract (TropicPure™willow bark extract with 15-20% Salicin; a registered trademark of Frutarom GmbH,Germany)
6.0 g cetyl alkohol
4.0 g glycerol mono stearate 60
7.5 g medium-chain triglycerides
25.5 g white vaseline
7.0 g macrogol-1000- glycerol mono stearate
10.0 g propylene glycole
35.0 g purified water

### 0.1% amarogentin nasal spray

100 µg/ml amarogentin in 2.5 % ethanol is dissolved in 0.9 % sodium chloride.
Per 20 ml: 200 µl amarogentin (10 mg/ml dissolved in 100% ethanol)
   300 µl 100% ethanol
   19.5 ml 0.9% sodium chloride

### 0.1% diphenidol nasal spray

100 µg/ml diphenidol in 2.5 % ethanol is dissolved in 0.9 % sodium chloride
Per 20 ml: 200 µl diphenidol (10 mg/ml dissolved in 0.9 % sodium chloride)
   500 µl 100% ethanol
   19.5 ml 0.9% sodium chloride
Instead of sodium chloride sea salt may be used.

In addition, hyaluronic acid may be added at a concentration of from 0.1% to 10%, preferably 0.5% to 2%, e.g. about 1%.

### Therapeutic uses

The invention further pertains to the therapeutic and/or prophylactic use of a ligand to a bitter taste receptor described herein. Said ligand can be used for the prevention and/or treatment of skin disorders.

Skin disorders include one of the following, but are not limited to, atopic dermatitis (neurodermatitis), skin cancer, lupus, rubeola, acne, hemangioma of skin, cold sore, psoriasis, rosacea, seborrhoic eczema, hives, vitiligo, warts, necrotizing fasciitis, cutaneous candidiasis, carbuncle, cellulitis, hyperhidrosis, impetigo, cutis laxa, decubitus ulcer, erysipelas, diaper rush, dyshydrotic eczema, canker sore, herpes stomatitis, fungal nail infection, ichthyosis vulgaris, dermatomyositis, ingrown nails, acrodermatitis, seborrhoic cyst, ceboreic ceratosis, pilonidel sinus, keloid, lichen planus, actinic keratosis, stasis dermatitis and leg ulcers, corns and calluses, eczema, tinea versicolor, pemphigoid, mouth ulcers and shingles. In one embodiment the skin disorder is an allergic skin disorder. In another embodiment the skin disorder is an inflammatory skin disorder. In yet another embodiment the skin disorder is an inflammatory and allergic skin disorder.

In a specific embodiment said skin disorder is pruritus, urticaria, dermatitis or eczema. In a preferred embodiment the skin disorder is neurodermitis.

The ligand described herein is administered topically to the patient. The ligand is administered topically to the skin of a patient, e.g. in the form of an ointment, a cream, lotion, or gel. The ligand can be administered to the skin of the patient at a dose from once daily to four times daily, preferably at a dose two to three times daily, more preferably three times daily.

One advantage of the present invention is that there is a very quick relief in the patients treated. Preferably, itching is reduced on a visual analog scale by at least 10%, preferably by at least 25%, more preferably by at least 50% within 24 hours after administration of a pharmaceutically effective dose of the ligand or of the pharmaceutical composition of the present invention.

In the case of skin inflammation, i.e. atopic dermatitis, skin redness is reduced on a visual analog scale by at least 10%, preferably by at least 25%, more preferably by at least 50% within 24 hours after administration of a pharmaceutically effective dose of the ligand or of the pharmaceutical composition of the present invention.

This invention can be further illustrated by the following examples of preferred embodiments thereof, although it will be understood that these examples are included merely for purpose of illustration.

### EXAMPLES

### EXAMPLE 1

### Materials and Methods

### Antibodies and reagents

The following antibodies and dilutions were used for immunohistochemical stainings: the rabbit polyclonal human anti-T2R1 antibody (Osensis, Thermo Fisher Scientific GmbH, Schwerte, Germany), 1:1000; the rabbit polyclonal human anti-S100 antibody (Dako,Glostrup, Denmark), 1:200; the polyclonal rabbit human anti-T2R38 antibody (Osenses), 1:750; the polyclonal rabbit human anti-T2R38 antibody (Abcam, Cambridge,UK), 1:1000, the goat polyclonal human anti-α-gustducin antibody (Santa Cruz Biotechnology, Heidelberg, Germany), 1:200; the rabbit polyclonal human anti-T1R2 antibody (Santa Cruz Biotechnology), 1:200 and the rabbit immunglobulin fraction (Dako) as negative control. The secondary antibody multi-link-biotin, the streptavidin-HRP-label and the AEC-substrate were from Dako and were used according to the manufacturer's instruction. The Alexa 488 donkey anti-goat and the Alexa 555 donkey anti-rabbit antibodies were from Invitrogen (Darmstadt, Germany) and used at a dilution of 1:500. The AccuMAx Array (Biocat, Heidelberg, Germany) contains 45 human tissues from various healthy organs with 2 spots of each case. The diameter of each spot on the slide was 1.0 mm. Each tissue section was extracted from various donor blocks and transferred into a ready-made recipient block. The donor blocks were formalin-fixed and paraffin-embedded. The human tongue whole tissue lysate (adult normal) was obtained from Novus biological (Cambridge, UK) and the human RNA from Agilent Technologies (Böblingen, Germany).

### Immunohistochemistry

Sections of the arrays were deparaffinized and subsequently subjected to a 20-min cooking pretreatment in target retrieval solution (pH 6.0; Dako) or proteinase K (Dako) treatment. Immunostaining was performed with the anti- T2R1 and -38, anti-α-gustducin, anti-S100 and anti-T1R2 antibody. Application of the primary antibody (4°C, overnight) was followed by incubation with biotinylated swine, anti-goat, anti-mouse and anti- rabbit antibody immunoglobulins (1 h, RT), streptavidin conjugated to horseradish peroxidase (20 min, RT), AEC solution as chromogen and hematoxylin counterstaining. Stainings with the rabbit immunglobulin fraction served as isotype control. The tissue arrays were scanned with the MIRAX scanner (Zeiss, Jena, Germany) and the staining was evaluated according to anatomical structures.

### Immunofluorescence

To perform T2R1 or T2R38 and α-gustducin double labeling, the cells were stained with the polyclonal rabbit human anti-T2R1 or T2R38 antibody and the goat human anti-α-gustducin antibody. The required secondary antibodies (Alexa 488 donkey anti-goat and Alexa 555 donkey anti-rabbit antibodies) were applied for 2 h at RT according to the manufacturer's instructions. The cells were then stained with DAPI and mounted in fluorescence mounting medium (Dako). Images were taken with a fluorescence microscope (Zeiss) equipped with the Axiovision software.

### Cell culture

The human keratinocyte cell line HaCaT, the neuroblastoma cell line SH-SY5Y and the placental cell line JEG-3 were from CLS Cell Lines Service (Heidelberg, Germany) and cultured in Dulbecco's modified essential medium (DMEM; Invitrogen) containing 10% fetal calf serum (FCS; PAA, Pasching, Austria) at 37 °C in a humidified atmosphere with 5% CO₂. For double fluorescence stainings the cells were seeded in a four-field chamber slides (Thermo Fisher Scientific GmbH; 1×10⁵ cells/ml). For calcium imaging experiments, cells were seeded on polylysin (Sigma-Aldrich, Munich, Germany) coated cover slips at a density of 1.5 -2x 10⁵. After isolation of human primary keratinocytes (hPK) from skin samples, the cells were incubated for 3 days with calcium (2 mM), amarogentin (100 µM) or diphenidol (100 µM) before RNA isolation and determination of the differentiation markers.

### Cytotoxicity test

Cytotoxicity of amarogentin in HaCaT cells was assessed with the ViaLight Plus ATP assay (Cambrex, Verviers,Belgium) according to the manual instructions. The method is based on the bioluminescence measurement of ATP that is present in metabolically active cells. Luciferase catalyzes the formation of light from ATP and luciferin. The emitted light intensity is directly proportional to the ATP concentration and is measured using a luminometer (Sirius HT; MWG).

### Western blot

Cell lysates were prepared from HaCaT, JEG-3 and SH-SY5Y cells and Western blot analysis was performed according to standard procedures. For antibody pre-absorption experiments the anti-T2R38 antiserum was pre-incubated with a 5-fold excess (v/v) of the immunogenic peptide for 1 h at 37°C. Then the antibody was applied as primary antibody to the blotting membrane and the normal western blot procedure was continued.

### RNA extraction and PCR

Total RNA was extracted with the RNeasy Mini kit (Quiagen, Hilden, Germany) from subconfluent HaCaT, JEG-3 and SH-SY5Y cells. First-strand cDNA was synthesized from 2 µg total RNA in 20 µl final volume using the Omniscript kit (Qiagen, Hilden, Germany) with random hexamer primers (Invitrogen). 2 µl aliquots of the reverse transcription solution were used as a template for specific PCR reactions with an annealing temperature of 58°C and the PCR product was analyzed by gel electrophoresis. The PCR primers (20 pmol each) used to amplify T2R1, T2R38, T1R1 and the house keeping gene ß-actine were:
T2R1 forward primer: 5'- gatctcctt ctttcttgtc tg- 3', reverse primer: 5'- taaaattaagatgagagagtg-3'; T2R38 forward primer: 5'-cgcatccgcactgtgtccta-3'; reverse primer: 5'-gggaatctgccttgtggtcg-3'; T1R3 forward primer: 5'-gcaagttctt cagcttcttcct-3', reverse primer: 5'-gtacatgttctccaggagctgc -3' and ß-actine forward primer: 5'-ccccaggcaccagggcgtgat -3'; reverse primer: 5'-ggtcatcttctcgcggttggccttggggt -3'.

### Restriction Enzyme-Based Detection of the T2R38 gene allele to distinguish between PTC taster and non-taster:

The most common single nucleotide polymorphisms (SNPs) in the T2R38 are in protein positions 49, 262 and 296. PTC tasters have the haplotype PAV and non-tasters have the haplotype AVI (A=Ala, V=Val, I=Ile). The sequence differences at the first and middle positions can be used to distinguish PAV tasters and AVI non-tasters by using a restriction enzyme that overlaps these two regions and so differentially cuts DNA depending upon the allele. First a 1067 bp fragment of the N-terminal portion of T2R38 is amplified by PCR (forward primer 5'catccctctaagtttcctgccaga, reverse primer 5'ttgggataatggcagcttgtccctc , annealing temperature: 58°C). Then the PCR product was extracted from a 0.8% agarose gel with the gel extraction kit from Qiagen and then cut with Fnu4H (New England Biolabs GmbH, Frankfurt) at 36°C for 15 minutes. Fnu4H cuts DNA at the sequence GCNGC (N is A, G, C or T). This sequence overlaps both polymorphism sites. At the amino acid position 262, PAV tasters contain the Fnu4H recognition sequence (G**C**TGC) while AVI non-tasters do not (G**T**TGC). At the amino acid position 49, PAV tasters contain also the Fnu4H cutting site (GCAG**C**) while AVI non-tasters do not (GCAG**G**). The restriction pattern of the T2R38 PCR product indicates the sequence at both polymorphic sites and was determined in a 2% agarose gel. A 557bp Fnu4H fragment in non-tasters is cleaved to a 456 bp and a75 bp fragment in PAV tasters. A 363 bp Fnu4H fragment in non-tasters is cleaved to a 336 bp and a27 bp fragment in PAV tasters (see Figure 9A for a schematically description of the cleaving products). Heterozygosity displays both the cleaved and un-cleaved products.

### Fluorescence Measurements

Intracellular Ca²⁺ concentration measurements in single cells were carried out using the fluorescence indicator fura-2-am (Invitrogen). Cells were washed with a buffer containing 130 mM NaCl, 5 mM KCI, 10 mM HEPES, 10 mM Glucose and 1 mM CaCl₂ adjusted to pH 7.4 and loaded with 2 µM fura-2-am and 0.04% Pluronic F-127 (Invitrogen) for 30 min. After rinsing twice, cells were allowed to de-esterify fura-2-am for 30 min. Measurements were performed with a monochromator-based imaging system attached to an inverted microscope (Axiovert S100, Zeiss). Fluorescence was excited at 340 and 380 nm and emission measured at 510 nm. After 60s, cells were stimulated with various concentrations of diphenidol or amarogentin, 100 µM PTC or the respective solvent control. T2R38 receptor inhibition was performed by pre-incubation of JEG-3 cells for 1h with the T2R38 antagonist probenecid (1 mM) before PTC stimulation. After correction of background fluorescence, the fluorescence ratio was calculated using Axiovision software.

### Reverse transcription PCR

Reverse transcription PCR was performed as described (Muller et al.,2008). In brief, total RNA was isolated using the Trizol reagent (Invitrogen) according to the manusfacturer's instruction. First-strand cDNA was synthesized from 2 mg total RNA in 20 ml final volume using the Omniscript kit (Qiagen, Hilden, Germany) with random hexamer primers (Invitrogen). 2 ml aliquots of the reverse transcription solution were used as a template for specific PCR. The PCR primers (20 pmol each) used to amplify keratin 10 (KRT10), INV, TGM and ß-actine were already published (Muller et al., 2008). ß-actine was used as internal loading control. After gel electrophoresis quantification was archived with ImageJ software.

### Results

### Strong T2R and α-gustducin expression in human tissue

Immunohistochemical stainings were performed to determine the expression of the two conserved bitter taste receptors T2R1 and T2R38 in 45 human tissues that were provided on a tissue microarray. T2R1 is the only taste receptor that is located on chromosome 5 and T2R38 is the best characterized bitter taste receptor that is located on chromosome 7 and closely related to the other T2Rs. Furthermore the tissue microarray was stained against the taste-specific G-protein α-gustducin to identify tissues that show no expression for T2R1 and T2R38 but possibly for another of the 25 described human bitter taste receptors. The specificity of the used anti-T2R38 antibody from Abcam was recently described by immunocytochemical experiments in which the anti-T2R38 antiserum was able to detect the corresponding receptor in transiently transfected mammalian cells [17]. In control sections, no staining was seen with an isotype control antibody. The staining results with the anti-T2R38 antibody from Abcam were identical with the anti-T2R38 antibody from Osenses (data not shown) so that this antibody also reacts specifically with the respective T2R38 antigen. This is especially important as Behrens and colleagues tested many antisera and several failed to recognize their specific epitope. The specificity of the antibody was also proven by western blot experiments with human normal tongue lysates (see tongue tissue as positive control in Figure 6B). All staining results were summarized in Table 1.

Four major tissue types (brain, skin, placenta and mucous epithelia of gland ductus) were found to express bitter taste receptors outside of the gustatory epithelia. To verify that this is an exclusive expression of bitter taste receptors the results were compared with stainings against the T1R2 sweet taste receptor.

### 1. Expression of T2R1, T2R38 and α-gustducin in human brain tissue

The immunohistochemical analysis showed strong expression of T2R1 and T2R38 in pyramidal neurons in the cerebral cortex, whereas glial cells (e.g. astrocytes, oligodendrocytes) did not express T2R1 and T2R38 (Figure 1B-C). The same staining pattern could be seen with an anti-α-gustducin antibody (Figure 1D). In the cerebellum only Purkinje neurons were bitter taste receptor and α-gustducin positive whereas the granule cells remained unstained (Figure 1G-I). The Purkinje neurons regulate and coordinate motor movements and lie in a row in the cerebellum between the molecular layer, a region relatively free of neuronal somata with the projected axons of granule cells, and the granule cell layer. Interestingly neither pyramidal neurons nor Purkinje neurons express the T1R2 sweet taste receptor (Figure 1E, J). No staining was seen in the cerebral cortex and cerebellum tissue with an isotype control antibody (Figure 1A, F). Furthermore the basal ganglia, a collection of nuclei situated at the base of the forebrain as well as the spinal cord, a thin, tubular bundle of nervous tissue that support cells that extends from the brain, did not express the analyzed bitter taste receptors and α-gustducin (data not shown).

### 2. Expression of T2R1, T2R38 and α-gustducin in the human epidermis

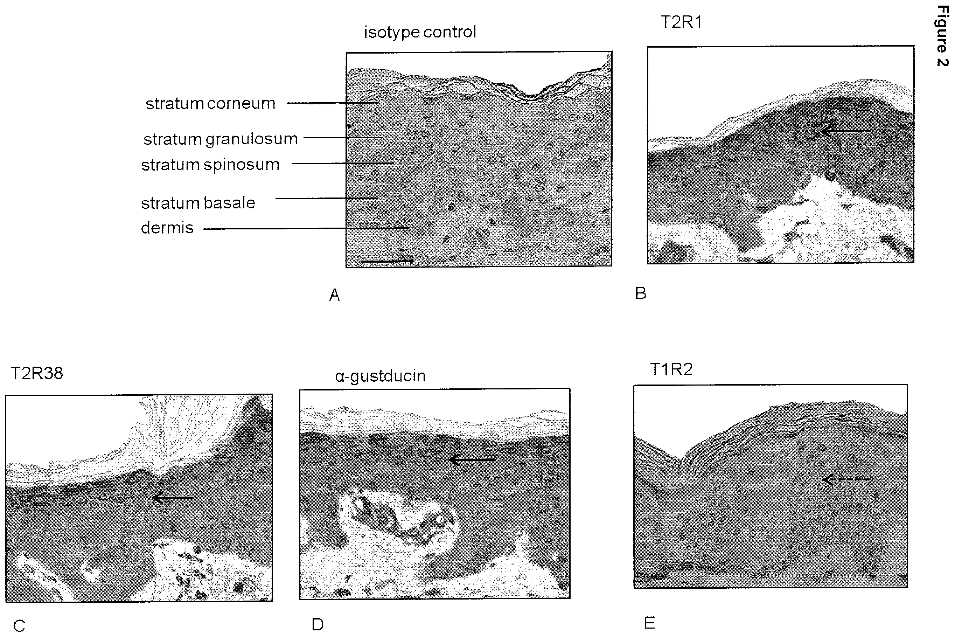

The epidermis, the outermost layer of the skin, is a stratified keratinized epithelium and is mostly composed of keratinocytes (proliferating basal and differentiating suprabasal keratinocytes). Some of the basal keratinocytes, of which some are epidermal stem cells, showed only a weak T2R1, T2R38 and α-gustducin expression (Figure 2B-D). During the differentiation process keratinocytes withdraw from the cell cycle, initiate expression of epidermal differentiation markers (e.g. keratin 1, involucrin) and move suprabasally where they become part of the stratum spinosum, stratum granulosum and eventually become dead corneocytes in the stratum corneum. Corneocytes have completed their differentiation program, lost their nucleus and cytoplasmic organelles. During this differentiation process keratinocytes from the stratum spinosum to the stratum corneum strongly expressed these two bitter taste receptors and α-gustducin (Figure 2B-D). To test if sweet taste receptors were also expressed in keratinocytes skin tissue was stained against T1R2. Only a slight T1R2 expression was detected in the epidermis and this expression increased only a little during the differentiation process of the keratinocytes (Figure 2E). No staining was seen in the human epidermis with an isotype control antibody (Figure 2A).

### 3. Expression of T2R1, T2R38 and α-gustducin in the human placenta

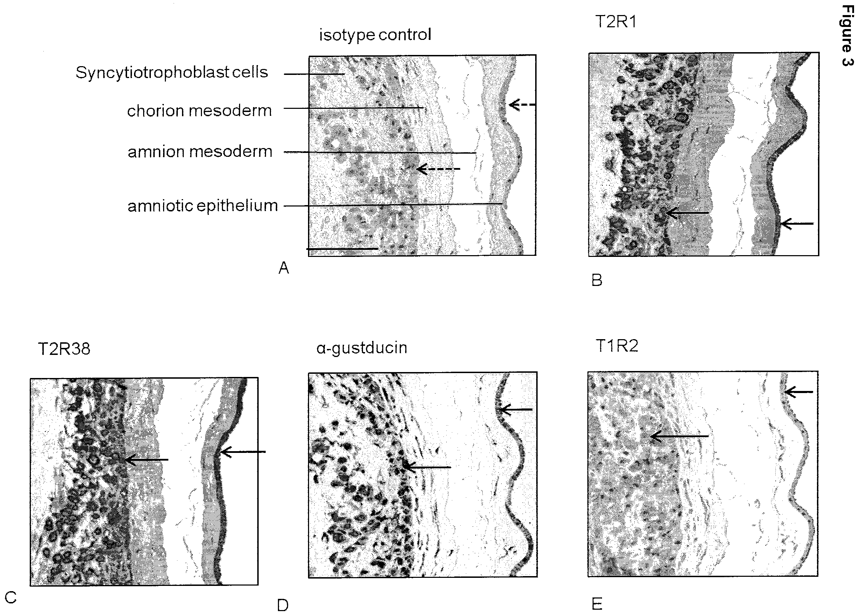

The placenta is a temporary organ required for the development of the embryo and fetus. It allows the exchange of metabolic products between the fetus and the mother. The placenta functions as a fetomaternal organ with two components: the fetal placenta (chorionic plate) and the maternal placenta (the basal plate). The fetal chorionic plate is composed of amnion, chorion and syncytiotrophoblast. The latter is an external layer that forms cords which invade the wall of the uterus to establish nutrient circulation between the embryo and the mother. The amnion epithelium (a membrane that surrounds and protects the embryo) and the syncytiotrophoblast showed a strong T2R1, T2R38 and α-gustducin staining (Figure 3B-D). In contrast, the T1R2 expression of the chorionic plate was only weak (Figure 3E). The basal plate (maternal placenta), the part of the uterus to which the villi anchor, stained only weakly for T2R1 and 38 (data not shown). No staining was seen in the human placenta with an isotype control antibody (Figure 3A).

### 4. Expression of T2R1, T2R38 and α-gustducin in some human glands

Another cell type that strongly expressed T2R1 and T2R38 was the simple columnar epithelium of secretory intralobular ducts (Figure 4B-C, red arrows). Serous and mucous acini were nearly unstained, for example in the parotid gland (Figure 4B-C, black arrows). These columnar epithelial cells expressed only weakly α-gustducin and were almost negative for T1R2 expression (data not shown). Furthermore, spongiocytes of the zona fasciculata of the adrenal gland cortex, straight tubular intestinal ducts of the colon (Figure 4E-F) and collecting tubules of the renal medulla showed a strong T2R1 and T2R38 staining. In the kidney cortex only the distal tubules were positive for bitter taste receptors (Table 1). No staining was seen in the parotid gland and ducts of the colon with an isotype control antibody (Figure 4A, F).

### T2R1, T2R38 and α-gustducin expressing single cells in human tissues

The pancreas is a bifunctional gland, having features of both an endocrine gland in the cell cluster of insulin secreting Langerhans cells and an exocrine gland that secretes digestive enzymes. The exocrine secretory acini in the pancreas expressed only very weakly T2R1, T2R38 and α-gustducin; however, there were some cells with strong bitter taste receptor-expression detectable in the pancreas parenchyma (data not shown). For better characterization of these cells, S100 staining was performed to determine if the T2R1-positive cells were of myoepithelial or neuronal origin or dendritic cells. The S-100 staining of the pancreas showed single S100-positive cells that are morphologically equal to the bitter taste receptor positive cells. Although S100 is not specific for dendritic cells and may also be expressed by neuronal and myoepithelial cells [19], S100 positive cells with dendritic morphology (oval nuclei, elongated cell size with few cytoplasm) and their location within the epithelium of ducts and acini in addition to the interstitial connective tissue, strongly suggests that these cells are in fact dendritic cells similar to the recently described cells in the salivary glands [20]. A few S100, T2R1, T2R38 and α-gustducin- positive cells could also be seen in simple columnar secretory epithelium lining the endocervical glands (Figure 5B-E). The S100-positive cells might be myoepithelial cells that are contractile and aid in secretion. The S100-positive cells in the pancreas and endocervix were not T1R2-positive (Figure 5F), in contrast to the acinar cells of the pancreas that expressed T1R2. No staining was seen in the endocervical gland with an isotype control antibody (Figure 5A). The stratified squamous epithelium of the ectocervix was strongly positive for bitter taste receptor expression (Table 1).

### Absent T2R1, T2R38 and α-gustducin expression in human tissues

Several human mesodermal tissues show neither a staining against T2R1, T2R38 and α-gustducin nor T1R2. These tissues include lymphoid tissues such as lymph nodes (supplementary data, not shown), thymus, spleen as well as heart, skeletal muscle, soft tissue, myometrium and ovary (Table 1).

**Table 1**

| Summary of T2R1, T2R38 and α-gustducin-positive and -negative human tissues. | | | |
|---|---|---|---|
| **blastodermic layer** | **Organ system** | **cells/tissues** | **Gustducin and T2R1/ T2R38 expression** |
| ectodermal | central nervous system | neuronal cells in: | positive |
| | | cerebral cortex, cerebellum, basal ganglion, hippocampus, spinal cord | |
| ectodermal | skin | keratinocytes fibroblasts mucous membrane (tongue, palate) | positive |
| ectodermal | placenta | placenta amnion | positive |
| | | placenta chordivilli, placenta basal | |
| | | umbilical cord | |
| ectodermal | gastrointestinal mucosa and ducts | mucous epithelial cells and gland ductus of: | |
| | | ileum, cecum, colon, rectum, parotid gland, kidney, breast, esophagus, stomach | positive |
| | | | negative |
| | | pancreas | negative, only a few cells are positive (see in the result section) |
| endodermal | urogenital system | prostate, testis, exocervix, endocervix | positive |
| | | | single cells positive |
| | | pro-endometrium | positive |
| | | sec-endometrium | |
| | | ureter | |
| | | myometrium, ovary, fallopian | |
| | | tube | negative |
| | | liver | |
| endodermal | respiratory ducts | lung | negative |
| mesodermal | mesenchymal structures | skeletal muscles, fat tissue, soft tissue, heart, endothelial cells | negative |
| mesodermal | lymphatic tissue | lymph node, spleen, tonsils, thymus | negative |

### RNA and protein expression of T2R1, T2R38 and T2R3 in human cell lines

The expression of T2R1, T2R38 and T1R3 was determined in three human cell lines corresponding to tissues that strongly expressed T2R1 and T2R38 i.e. the human neuroblastoma cell line SH-SY5Y, the placental cell line JEG-3 and the keratinocyte cell line HaCaT (Figure 6A) by evaluating the expression of their mRNA using RT-PCR. Commercially available tongue RNA was used as positive control. The expression of the house keeping gene ß-actine demonstrated equal RNA content. All cell lines and the tongue tissue expressed T2R1 and T2R38, however only the tongue tissue expressed T1R3.

The T2R1, T2R38 and T1R3 gene expression results were confirmed on the protein level by western blot with protein lysates from the three cell lines and the positive control tongue tissue (Figure 6B). As no human anti-T1R3 antibody, involved in both sweet and umami taste perception, was available, we used an antibody against the sweet taste receptor T1R2. The detection of HSC-70 was used as loading control (Figure 6B, last picture). The bands correspond to the predicted molecular weight of receptor monomers, glycosylated monomers and oligomers. It has been observed that T2Rs form homo- and heteromers *in vitro.* Although a physiological significance of the receptor hetero-oligomerization was not obvious, receptor oligomerization might in general be necessary for receptor function [21]. Furthermore hT2Rs contain a conserved N-glycosylation site in the 2^{nd} extracellular loop, so the band migrating just above 50 kDa in the Western blot likely corresponds to the full length N-glycosylated receptor monomer (Figure 6B, first picture) [17]. Asn (N)-linked glycolysation seems to be associated with folding and cell surface transport together with RTP (receptor transporting protein) and REEP (receptor expression enhancing protein) but not for its function per se [22] [23]. Furthermore slower migrating putative receptor oligomers as well as a faster migrating monomer or possibly truncated receptor variant are visible as already described [17]. The un-glycosylated monomers are only visible in the lysates from the cell lines but not in the tongue tissue lysate, because the T2R pattern is possibly different in tissue lysates as compared to cell line lysates. The specificity of the T2R38 antibody is demonstrated by parallel experiments in which the primary anti-T2R38 antiserum has been pre-absorbed with the corresponding antigenic peptides. As expected this antiserum preparation with reduced levels of reactive specific anti-T2R38 antibodies did not detect T2R38 in the cell lysates (Figure 6B, second picture). The bands of the western blot detected with an anti-T2R1 antibody correspond to the predicted molecular weight of receptor monomers, glycosylated monomers and oligomers as for the anti- T2R38 antibody (Figure 6, third picture). The T1R2 sweet taste receptor reveals a major immunoreactive band migrating at approximately 50 kDa, in agreement to the theoretically calculated molecular weight and data from the literature [24]. Only in the tongue tissue but not in the other cell lines are two bands in the Western blot detectable (Figure 6B, fourth picture). Furthermore the T1R2 forms heterodimers with the T1R3 receptor, so that the higher band might also represent the heterodimer.

### Coexpression of T2R1 or T2R38 with α-gustducin in human cell lines

To perform T2R1 or T2R38 and α-gustducin double labeling, the human neuroblastoma cell line SH-SY5Y (Figure 7A), the placental cell line JEG-3 (Figure 7B) and the keratinocyte cell line HaCaT (Figure 7C) were stained with the polyclonal rabbit human anti-T2R1 or T2R38 antibody and the goat human anti-α-gustducin antibody. Then the required secondary antibodies (Alexa 488 donkey anti-goat and Alexa 555 donkey anti-rabbit antibodies) were applied and the cells were then stained with DAPI. It could be demonstrated that the T2R1 and T2R38-positive neuronal and placental cells as well as keratinocytes expressed α-gustducin. No fluorescence signal was detectable with an isotype control antibody (Figure 7A-C first picture).

### The bitter compound diphenidol induces calcium influx in a human neuroblastoma, a human placental and a human epithelial cell line

Having demonstrated that the neuroblastoma cell line SH-SY5Y, the human placental cell line JEG-3 and human keratinocyte cell line HaCaT express α-gustducin as well as the bitter taste receptors T2R1 and T2R38, the next step was to determine if the expressed bitter taste receptors are functional by measuring intracellular calcium influx after stimulation with diphenidol (DPH). Diphenidol is a synthetic bitter taste receptor agonist which activates 16 bitter taste receptors including T2R1 and T2R38 as described [25]. DPH medication has long been clinically developed as an antiemetic and an antivertigo agent [26]. First we stimulated HaCaT cells with increasing amounts of diphenidol and noticed already with 30 µM a significant calcium influx (Figure 11). According to data from the literature all cell lines under investigation were stimulated with 100 µM diphenidol and showed a significant calcium influx (up to 7-fold) compared to the solvent control (Figure 8). However, diphenidol is a muscarinic receptor antagonist and therefore not specific for T2Rs. Diphenidol is also described as an inhibitor of voltage gated K⁺ and Ca²⁺ channels without affecting store-operated Ca²⁺ channels. But the precise cellular and molecular action of diphenidol is still not fully clear [26]. Therefore we performed additional activation experiments in HaCaT cells with different concentrations of amarogentin, the bitterest substance in the nature that is present in higher amounts in *Gentiana lutea* and can activate several T2Rs (i.e. T2R1, 4, 39, 43, 46, 47 and 50) to prove the functionality of the T2Rs. A concentration of 100 µM amarogentin led to a significant calcium influx in HaCaT cells (Figure 11).

### PTC induced calcium influx in the taster cell line JEG-3

Next we wanted to stimulate the three cell lines with a substance that only activates T2R1 or T2R38 to provide a strong proof for the T2R function in these non-gustatory tissues. PTC (Phenylthiocarbamid) is a good substance for this purpose, because it specifically activates T2R38 in taster variants (PAV haplotype). First we determined the T2R38 variant in our three analyzed cell lines by restriction enzyme analyses (Figure 9 B) and gene sequencing (Figure 9C) of a 1067 bp fragment of the N-terminal portion of T2R38. The pattern of the cleaving products after digestion with the restriction enzyme Fnu4H allows the distinction between PAV tasters and AVI non-tasters (see Figure 9A and Material and Method section). The DNA of the placental cell line JEG-3 contains the taster variant (PAV), the keratinocyte cell line HaCaT is heterozygous (PAV/AVI) and the DNA of SH-SY5Y cells contains the homozygous non-taster variant (AVI). Therefore we used the JEG-3 cells for PTC activation analyses. PTC induced calcium influx in JEG-3 cells. Our data are comparable to the calcium influx results described by Greene and colleagues [18], who worked with HEK293 cells transfected with T2R38 DNA before PTC stimulation and calcium influx measurement. To further demonstrate the specificity of the PTC activation in JEG-3 cells this activation was repressed with the T2R38 inhibitor probenecid as described by Greene and colleagues (Figure 10a and B). These results in JEG-3 cells demonstrate a very specific T2R38 activation.

### Induction of differentiation by amarogentin and diphenidol

As calcium influx is involved in differentiation we investigated if amarogentin or diphenidol could induce differentiation in primary keratinocytes by analyzing the expression of the differentiation marker keratin 10, involucrin und transglutaminase 3 days after stimulation with diphenidol, amarogentin or calcium.. The expression of all these markers could be increased (Figure 12). The used concentration of amarogentin and diphenidol showed no cytotoxicityin HaCaT cells (Figure 13).

### EXAMPLE 2

### Material and Methods

### Cell culture

LAD-2 human mast cells (obtained from Dr. A. Kirshenbaum NIH) were cultured in serum-free media (StemPro-34, Gibco, Germany) supplemented with 2mM L-glutamine and 100 ng/ml rhSCF (recombinant human stem cell factor; Cell signaling technologies). The human keratinocyte cell line HaCaT was cultured in Dulbecco's modified essential medium (DMEM; Invitrogen) containing 10% fetal calf serum (FCS; PAA, Pasching, Austria) at 37 °C in a humidified atmosphere with 5% CO₂.

### Human mast cell stimulation

LAD-2 cells were washed with PBS and resuspended in complete medium. LAD-2 cells (2x10⁵ cells /400 µl/well) were plated in 48 well flat bottom Falcon cell culture plates from Becton Dickinson and then stimulated with SP (2 µM, Sigma-Aldrich) for 1 or 24 h. Some cells were pre-incubated for 30 min with amarogentin (136 µM, Phytolab), diphenidol (136 µM, Santa Cruz) or azelastine (24 µM, Sigma) as indicated. The supernatants were collected for further assays.

### Human Keratinocyte stimulation

The Keratinocyte cell line HaCaT (2x10⁵ cells /400 µl/well) were plated in 48 well flat bottom Falcon cell culture plates from Becton Dickinson and then stimulated with histamine (100 µl) and TNF-α (25 ng/ml) or IFNγ (200U/ml) for 24 h. Some cells were pre-incubated for 30 min with amarogentin (136 µM, Phytolab), diphenidol (136 µM, Santa Cruz) or azelastine (24 µM, Sigma) as indicated. The supernatants were collected for further assays.

### Degranulation assays

Mast cell degranulation was assessed by measuring histamine in the supernatant fluid 1 h after cell stimulation with SP (2 µM). Histamine levels were assayed using a Histamine ELISA from IBL international.

### Cytokine and MMP-1 release assays

TNF-α, IL-6, IL-8 and MMP-1 release into the supernatant fluid 24 h after cell stimulation (either LAD-2 oder HaCaT cells) were measured by Enzyme-Linked Immunosorbent Assay (ELISA) using a commercial kit from R&D Systems according to the manual instructions.

### Cytotoxicity test:

Cytotoxicity of amarogentin and azelastine in LAD-2 and HaCaT cells was assessed with the ViaLight Plus ATP assay (Cambrex, Verviers,Belgium) according to the manual instructions. The method is based on the bioluminescence measurement of ATP that is present in metabolically active cells. Luciferase catalyzes the formation of light from ATP and luciferin. The emitted light intensity is directly proportional to the ATP concentration and is measured using a luminometer (Sirius HT; MWG).

### Results

### Amarogentin reduces histamine release in mast cells after SP-stimulation

LAD-2 mast cells release histamine 1 h after SP (2 µM) stimulation. Amarogentin (136 µM, 30 min) inhibits histamine release as well as the control substance azelastine (24 µM, 30 min) (Figure 14A). LAD-2 mast cells also secrete newly-synthesized TNF-α after SP (2 µM) stimulation. Pre-incubation with amarogentin (136 µM, 30 min) or diphenidol (136 µM, 30 min) as with the positive control azelastine (24 µM, 30 min) blocked the secretion of TNF-α from mast cells (Figure 14B).

Amarogentin reduces the release of IL-6, IL-8 and MMP-1 in keratinocytes treated with histamine together with TNF-α or IFN-γ. TNF-α stimulation caused in the keratinocyte cell line HaCaT the release of the cytokine IL-8 and the matrix metalloproteinase MMP-1. TNF-α increased the secretion of IL-8 and MMP-1, although histamine alone only marginally enhanced the production of IL-8 and MMP-1 (Figure 15A). IL-8 is an inflammatory cytokine that can chemoattract neutrophils and T cells and in this way initiates cutaneous inflammation. MMP-1 leads to increased T-cell transmigration through the basement membrane. Furthermore INF-γ together with histamine can induce HaCaT cells to produce the proinflammatory cytokine IL-6 and to produce also MMP-1. IL-6 and MMP-1 production can be decreased by amarogentin and the control azelastine (Figure 15B). The used concentration of amarogentin was not cytotoxic for HaCaT cells (Figure 16). A simplified working hypothesis for the action of the bitter taste receptor ligands is shown in Figure 17.

### References

1. Yarmolinsky DA, Zuker CS, Ryba NJ (2009) Common sense about taste: from mammals to insects. Cell 139: 234-244.
2. Nelson G, Hoon MA, Chandrashekar J, Zhang Y, Ryba NJ, et al. (2001) Mammalian sweet taste receptors. Cell 106: 381-390.
3. Adler E, Hoon MA, Mueller KL, Chandrashekar J, Ryba NJ, et al. (2000) A novel family of mammalian taste receptors. Cell 100: 693-702.
4. Chandrashekar J, Mueller KL, Hoon MA, Adler E, Feng L, et al. (2000) T2Rs function as bitter taste receptors. Cell 100: 703-711.
5. Matsunami H, Montmayeur JP, Buck LB (2000) A family of candidate taste receptors in human and mouse. Nature 404: 601-604.
6. Finger TE, Kinnamon SC (2011) Taste isn't just for taste buds anymore. F1000 Biol Rep 3: 20.
7. Callaway E (2012) Evolutionary biology: the lost appetites. Nature 486: S16-17.
8. Amrein H, Bray S (2003) Bitter-sweet solution in taste transduction. Cell 112: 283-284.
9. Lee RJ, Xiong G, Kofonow JM, Chen B, Lysenko A, et al. (2012) T2R38 taste receptor polymorphisms underlie susceptibility to upper respiratory infection. J Clin Invest 122: 4145-4159.
10. Kim UK, Drayna D (2005) Genetics of individual differences in bitter taste perception: lessons from the PTC gene. Clin Genet 67: 275-280.
11. Bufe B, Breslin PA, Kuhn C, Reed DR, Tharp CD, et al. (2005) The molecular basis of individual differences in phenylthiocarbamide and propylthiouracil bitterness perception. Curr Biol 15: 322-327.
12. Biarnes X, Marchiori A, Giorgetti A, Lanzara C, Gasparini P, et al. (2010) Insights into the binding of Phenyltiocarbamide (PTC) agonist to its target human TAS2R38 bitter receptor. PLoS One 5: e12394.
13. Shah AS, Ben-Shahar Y, Moninger TO, Kline JN, Welsh MJ (2009) Motile cilia of human airway epithelia are chemosensory. Science 325: 1131-1134.
14. Rozengurt E, Sternini C (2007) Taste receptor signaling in the mammalian gut. Curr Opin Pharmacol 7: 557-562.
15. Deshpande DA, Wang WC, Mcllmoyle EL, Robinett KS, Schillinger RM, et al. (2010) Bitter taste receptors on airway smooth muscle bronchodilate by localized calcium signaling and reverse obstruction. Nat Med 16: 1299-1304.
16. Lindemann B (2001) Receptors and transduction in taste. Nature 413: 219-225.
17. Behrens M, Born S, Redel U, Voigt N, Schuh V, et al. (2012) Immunohistochemical detection of TAS2R38 protein in human taste cells. PLoS One 7: e40304.
18. Greene TA, Alarcon S, Thomas A, Berdougo E, Doranz BJ, et al. (2011) Probenecid inhibits the human bitter taste receptor TAS2R16 and suppresses bitter perception of salicin. PLoS One 6: e20123.
19. Haimoto H, Hosoda S, Kato K (1987) Differential distribution of immunoreactive S100-alpha and S100-beta proteins in normal nonnervous human tissues. Lab Invest 57: 489-498.
20. Le A, Saverin M, Hand AR (2011) Distribution of dendritic cells in normal human salivary glands. Acta Histochem Cytochem 44: 165-173.
21. Kuhn C, Bufe B, Batram C, Meyerhof W (2010) Oligomerization of TAS2R bitter taste receptors. Chem Senses 35: 395-406.
22. Reichling C, Meyerhof W, Behrens M (2008) Functions of human bitter taste receptors depend on N-glycosylation. J Neurochem 106: 1138-1148.
23. Behrens M, Bartelt J, Reichling C, Winnig M, Kuhn C, et al. (2006) Members of RTP and REEP gene families influence functional bitter taste receptor expression. J Biol Chem 281: 20650-20659.
24. Raliou M, Grauso M, Hoffmann B, Schlegel-Le-Poupon C, Nespoulous C, et al. (2011) Human genetic polymorphisms in T1R1 and T1R3 taste receptor subunits affect their function. Chem Senses 36: 527-537.
25. Meyerhof W, Batram C, Kuhn C, Brockhoff A, Chudoba E, et al. (2010) The molecular receptive ranges of human TAS2R bitter taste receptors. Chem Senses 35: 157-170.
26. Leung YM, Wong KL, Cheng KS, Kuo CS, Su TH, et al. (2012) Inhibition of voltage-gated K+ channels and Ca2+ channels by diphenidol. Pharmacol Rep 64: 739-744.
27. Li F, Zhou M (2012) Depletion of bitter taste transduction leads to massive spermatid loss in transgenic mice. Mol Hum Reprod 18: 289-297.
28. Clark AA, Liggett SB, Munger SD (2012) Extraoral bitter taste receptors as mediators of off-target drug effects. FASEB J 26: 4827-4831.
29. Go Y, Investigators ST-NY (2006) Proceedings of the SMBE Tri-National Young Investigators' Workshop 2005. Lineage-specific expansions and contractions of the bitter taste receptor gene repertoire in vertebrates. Mol Biol Evol 23: 964-972.
30. Dong D, Jones G, Zhang S (2009) Dynamic evolution of bitter taste receptor genes in vertebrates. BMC Evol Biol 9: 12.
31. Leuner K, Kraus M, Woelfle U, Beschmann H, Harteneck C, et al. (2011) Reduced TRPC channel expression in psoriatic keratinocytes is associated with impaired differentiation and enhanced proliferation. PLoS One 6: e14716.
32. Dehkordi O, Rose JE, Fatemi M, Allard JS, Balan KV, et al. (2012) Neuronal expression of bitter taste receptors and downstream signaling molecules in the rat brainstem. Brain Res 1475: 1-10.
33. Upadhyaya J, Pydi SP, Singh N, Aluko RE, Chelikani P (2010) Bitter taste receptor T2R1 is activated by dipeptides and tripeptides. Biochem Biophys Res Commun 398: 331-335.
34. Witt M, Reutter K (1996) Embryonic and early fetal development of human taste buds: a transmission electron microscopical study. Anat Rec 246: 507-523.
35. Muller M, Essin K, Hill K, Beschmann H, Rubant S et al. (2008) Specific TRPC6 channel activation, a novel approach to stimulate keratinocyte differentiation J Biol.Chem., 283(49): 33942-54.

## Claims

1. A ligand to a bitter taste receptor (T2R) for use in the treatment of a skin disorder, wherein the ligand is administered topically to the skin, and wherein the ligand is amarogentin.

2. The ligand for use according to claim 1, wherein the bitter taste receptor is taste receptor 2 member 1 (T2R1).

3. The ligand for use according to claim 1, wherein said skin disorder is selected from the group consisting of pruritus, urticaria, dermatitis, eczema and combinations thereof.

4. A pharmaceutical composition for topical use in the treatment of a skin disorder, comprising a ligand to a bitter taste receptor as defined in claim 1, wherein said composition is an ointment, a lotion, a cream or a gel.

## Patentansprüche

1. Ligand für einen Bittergeschmacksrezeptor (T2R) zur Verwendung bei der Behandlung einer Hauterkrankung, wobei der Ligand topisch an die Haut verabreicht wird und wobei der Ligand Amarogentin ist.

2. Ligand zur Verwendung nach Anspruch 1, wobei der Bittergeschmacksrezeptor der Geschmacksrezeptor 2, Untereinheit 1 (T2R1) ist.

3. Ligand zur Verwendung nach Anspruch 1, wobei die Hauterkrankung aus der Gruppe ausgewählt ist, bestehend aus Pruritus, Urticaria, Dermatitis, Ekzem und Kombinationen davon.

4. Pharmazeutische Zusammensetzung für die topische Verwendung bei der Behandlung einer Hauterkrankung, umfassend einen Liganden für einen Bittergeschmacksrezeptor, wie in Anspruch 1 definiert, wobei die Zusammensetzung eine Salbe, eine Lotion, eine Creme oder ein Gel ist.

## Revendications

1. Ligand pour un récepteur de goût amer (T2R) pour utilisation dans le traitement d'un trouble de la peau, dans lequel le ligand est administré par voie topique à la peau et dans lequel le ligand est l'amarogentine.

2. Ligand pour utilisation selon la revendication 1, dans lequel le récepteur de goût amer est le récepteur de goût 2 élément 1 (T2R1).

3. Ligand pour utilisation selon la revendication 1, dans lequel ledit trouble de la peau est choisi dans le groupe constitué du prurit, de l'urticaire, de l'eczéma et de leurs combinaisons.

4. Composition pharmaceutique pour utilisation topique dans le traitement d'un trouble de la peau, comprenant un ligand pour un récepteur de goût amer selon la revendication 1, dans laquelle ladite composition est un onguent, une lotion, une crème ou un gel.
